# FASCICULE DE BREVET EUROPEEN

(11) **EP 0 857 052 B1**
(45) Date de publication et mention de la délivrance du brevet: **02.05.2001**
(21) Numéro de dépôt: 96934947.1
(22) Date de dépôt: 21.10.1996
(51) Int. Cl.: A61K 7/06, A61K 7/09, A61K 7/135

(54) **NOUVELLE COMPOSITION OXYDANTE ET NOUVEAU PROCEDE POUR LA DEFORMATION PERMANENTE OU LA DECOLORATION DES CHEVEUX**
NEUE OXIDIERENDE ZUSAMMENSETZUNG UND EIN NEUES VERFAHREN ZUR DAUERHAFTEN VERFORMUNG ODER ENTFÄRBUNG VON HAAREN
NOVEL OXIDISING COMPOSITION AND NOVEL METHOD FOR PERMING OR BLEACHING HAIR

(30) Priorité: 20.10.1995 FR 9512386
(43) Date de publication de la demande: 12.08.1998
(73) Titulaire: L'OREAL, 75008 Paris (FR)
(72) Inventeur: MAUBRU, Mireille, F-78400 Chatou (FR); BRAIDA-VALERIO, Damarys, F-75004 Paris (FR)
(74) Mandataire: Miszputen, Laurent
(86) Numéro de dépôt international: FR9601644
(87) Numéro de publication internationale: WO9715273

(56) Documents cités:
- EP-A- 0 647 617
- FR-A- 2 679 770

## Description

L'invention concerne une composition cosmétique oxydante, pour réaliser une déformation permanente ou une décoloration des cheveux et des procédés de traitement des matières kératiniques, en particulier des cheveux, en vue d'obtenir une déformation permanente de ces dernières, en particulier sous la forme de cheveux permanentés, ou une décoloration.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation terriporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses divers dérivés, la cystéamine et ses dérivés, l'acide thiolactique ou l'acide thioglycolique, leurs sels ainsi que leurs esters, notamment le thioglycolate de glycérol.

Concernant les compositions oxydantes nécessaires à la mise en oeuvre de l'étape de fixation, on fait le plus souvent appel, dans la pratique, à des compositions à base d'eau oxygénée ou de bromates alcalins.

Le problème de la technique des permanentes connues à ce jour est que leur application sur les cheveux induit à la longue une altération de la qualité des cheveux. Les causes essentielles de cette altération de la qualité des cheveux sont une diminution de leurs propriétés cosmétiques, telles que leur brillance, et une dégradation de leurs propriétés mécaniques, plus particulièrement une dégradation de leur résistance mécanique due à un gonflement des fibres kératiniques lors du rinçage entre l'étape de réduction et l'étape d'oxydation qui peut également se traduire par une augmentation de leur porosité.

Les cheveux sont affaiblis et peuvent devenir cassants lors de traitements ultérieurs comme des brushings.

On retrouve le même problème d'altération de la fibre kératinique lors des procédés de coloration ou de décoloration des cheveux. Ces derniers sont sensibilisés, c'est-à-dire abîmés à des degrés divers par les traitements capillaires, mécaniques ou chimiques, tels que les colorations, les décolorations et/ou les permanentes.

Pour résoudre ce problème d'altération de la qualité des cheveux, il a été proposé d'associer des polymères cationiques soit aux agents réducteurs, soit aux agents oxydants.

Mais ces solutions se révèlent insatisfaisantes dans la mesure où elles ne résolvent pas totalement le problème de la diminution des propriétés mécaniques des cheveux. En particulier, dans le cas d'un traitement de déformation permanente des cheveux, ces derniers présentent un toucher non satisfaisant et la tenue de la frisure est insuffisante.

La présente invention a notamment pour but de résoudre les problèmes ci-dessus.

Plus précisément, elle a pour but de proposer une nouvelle composition oxydante, qui, utilisée notamment lors du deuxième temps d'une opération de déformation permanente des cheveux permet de limiter, voire d'empêcher, la dégradation des propriétés mécaniques des matières kératiniques, et plus particulièrement la casse des cheveux, et d'obtenir une belle frisure résistante au brushing et de bonne tenue.

Elle a également pour but de proposer une composition oxydante telle que ci-dessus qui permette d'améliorer les propriétés cosmétiques, telles que la douceur et la facilité de démêlage, des fibres kératiniques lorsque celles-ci subissent en particulier un traitement de déformation permanente.

Enfin, la présente invention a pour but de proposer un nouveau procédé de déformation permanente des cheveux, utilisant la composition oxydante selon l'invention.

Il a été proposé dans les demandes EP-A-0 647 617 et FR A 2 673 179 au nom de la Demanderesse d'utiliser des céramides particuliers en association avec des lipides comme enveloppe de vésicules encapsulant des substances actives hydrosolubles, ces substances actives pouvant être, entre autres, des oxydants, pour protéger lesdites substances actives des différents agents d'altération et des composés réactifs qui peuvent être présents dans la composition.

Or, la demanderesse vient de découvrir de façon tout à fait surprenante que l'utilisation de composés de type céramide dans la composition oxydante d'un procédé de déformation permanente exempte de vésicules encapsulant un agent oxydant permettait d'obtenir un excellent état de la fibre capillaire au terme du procédé de permanente.

La présente invention a donc pour objet une nouvelle composition oxydante comprenant, dans un support cosmétique approprié, i) au moins un composé de type céramide et ii) au moins un agent oxydant choisi dans le groupe formé par l'eau oxygénée, les bromates alcalins, les persels ou les polythionates ou leurs mélanges, ledit composé de type céramide étant présent dans la composition à une teneur allant de 0,005 % à 10 % en poids par rapport au poids total de la composition, ladite composition étant exempte de vésicules contenant un agent oxydant.

La présente invention a également pour objet un nouveau procédé de traitement des matières kératiniques, en particulier des cheveux, en vue d'obtenir une déformation permanente de ces dernières, en particulier sous la forme de cheveux permanentés, ce procédé comprenant les étapes suivantes : (i) on applique sur la matière kératinique à traiter une composition réductrice, la matière kératinique étant mise sous tension mécanique avant, pendant, ou après ladite application, (ii) on rince éventuellement la matière kératinique, (iii) on applique sur la matière kératinique éventuellement rincée une composition oxydante telle que définie ci-dessus, (iv) on rince éventuellement à nouveau la matière kératinique.

Le procédé selon l'invention convient particulièrement bien à l'obtention d'une chevelure permanentée sans risque de dégradation de la fibre kératinique. En particulier, le procédé selon l'invention limite la casse des cheveux. On obtient une belle frisure homogène, ainsi qu'une meilleure tenue de la coiffure. Le toucher mouillé des cheveux traités selon le procédé de l'invention est agréable et le coiffage est facilité. La forme aquise par des cheveux ayant subi le traitement de déformation permanente selon l'invention présente en outre une bonne rémanence dans le temps au shampooing.

La présente invention a également pour objet un nouveau procédé de décoloration des matières kératiniques, en particulier des cheveux, comprenant les étapes suivantes : i) on applique sur la matière kératinique une composition oxydante selon l'invention, cette composition comprenant de préférence de l'eau oxygénée seule ou en présence de persels en milieu alcalin, ii) on rince la matière kératinique ainsi traitée.

D'autres caractéristiques, aspects et avantages de l'invention apparaîtront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toute matière kératinique en général, notamment cils, moustaches, poils, laine et autres.

Dans ce qui précède et ce qui suit, on entend par vésicules des sphérules lipidiques constituées de couches moléculaires organisées enfermant une phase aqueuse encapsulée, ces couches étant constituées d'au moins un composé de type céramide associé à au moins un autre composé lipidique.

Selon la présente invention, on entend, par composés de type céramide, les céramides et/ou les glycocéramides et/ou les pseudocéramides. Ils sont choisis de préférence parmi les molécules naturelles ou synthétiques répondant à la formule (I) suivante : 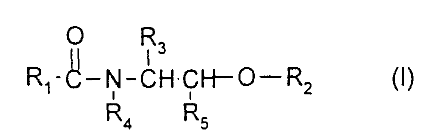 dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifiés par un acide gras saturé ou insaturé, linéaire ou ramifié, éventuellement mono ou polyhydroxylé, en C₁-C₃₅,
   - soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent,
   - soit un radical R₈-O-CO-(CH2)ₚ, R₈ désignant un radical hydrocarboné en C₁-C₂₀, p étant un entier variant de 1 à 12 ;
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄;
   de préférence, R₃ désigne un radical α-hydroxyalkyle en C₁₅-C₂₆, le groupement hydroxyle étant éventuellement estérifié par un α-hydroxyacide en C₁₆-C₃₀;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p étant un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (I) ci-dessus, on préfère les céramides et/ou glycocéramides décrits par DOWNING dans Journal of Lipid Research, Vol. 35, page 2060, 1994 ou ceux décrits dans la demande de brevet français FR-2 673 179.

Les composés de type céramide plus particulièrement préférés selon l'invention sont les composés de formule (I) pour lesquels R₁ désigne un radical alkyle saturé ou insaturé dérivé d'acides gras en C₁₄-C₂₂ éventuellement hydroxylé; R₂ désigne un atome d'hydrogène ; et R₃ désigne un radical linéaire saturé en C₁₁-C₁₇ éventuellement hydroxylé et de préférence en C₁₃-C₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (I) pour lesquels R₁ désigne un radical alkyle saturé ou insaturé dérivé d'acides gras ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂-CH₃.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés décrits dans les demandes de brevet EP-A-0 227 994, EP-A-0 647 617, EP-A-0 736 522 et WO 94 / 07 844.

De tels composés sont par exemple le QUESTAMIDE H, encore appelé bis-(N-hydroxyéthyl N-cétyl) malonamide et vendu par la société QUEST et le N-(2-hydroxyéthyl)-N-(3-cétyloxy-2-hydroxypropyl)amide d'acide cétylique .

On peut également utiliser le N-docosanoyl N-méthyl-D-glucamine tel que décrit dans la demande de brevet WO 94/24097.

De préférence, le composé de type céramide utilisé dans la présente invention est choisi parmi le 2-N-oléoylamino-octadécane-1,3-diol, le 2-N-[2-hydroxypalmitoyl]-amino-octadécane-1,3-diol et la N-stéaroylphytosphingosine.

Le ou les céramides et/ou glycocéramides sont présents dans la composition selon l'invention à une teneur allant de 0,005 % à 10 %, de préférence allant de 0,01 % à 5 %.

L'agent oxydant de la composition selon l'invention est choisi dans le groupe formé par l'eau oxygénée, les bromates alcalins, les persels ou les polythionates ou leurs mélanges, tel qu'un mélange de bromate alcalin et d'un persel, ou d'un persel et d'eau oxygénée. De préférence, l'agent oxydant des compositions selon l'invention est l'eau oxygénée.

La concentration en eau oxygénée peut varier de 0,5 à 40 volumes, de préférence de 2 à 30 volumes. La concentration en bromate alcalin est généralement de 1 à 12 % et celle en persel de 0,1 à 25 % en poids par rapport au poids total de la composition oxydante.

Le pH de l'ensemble de la composition oxydante est de préférence compris entre 1 et 13, et encore plus préférentiellement entre 2 et 12.

Ce pH peut être obtenu et/ou ajusté classiquement par ajout soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

La composition oxydante peut se présenter sous la forme d'une lotion, épaissie ou non, d'une crème, d'un gel ou de toute autre forme appropriée et peut contenir des additifs connus pour leur utilisation dans les compositions oxydantes pour la déformation permanente ou la décoloration des cheveux. Elle peut se présenter sous la forme d'un shampooing.

La composition oxydante peut également se présenter, en particulier dans le cas de la décoloration, sous forme de deux parties à mélanger au moment de l'emploi, l'une de ces deux parties contenant des agents alcalins et se présentant sous forme solide ou liquide.

La composition oxydante peut contenir en outre des additifs cosmétiques bien connus pour ce type de composition tels que des agents alcalinisants ou acidifiants, des agents conservateurs, des agents séquestrants, des cations, des opacifiants et éventuellement un polymère cationique.

Un deuxième objet de la présente invention est un procédé de déformation permanente des fibres kératiniques, et en particulier des cheveux, utilisant comme composition oxydante la composition définie ci-dessus.

La première étape de ce procédé consiste à appliquer sur les cheveux une composition réductrice. Cette application se fait mèche par mèche ou globalement.

La composition réductrice comprend au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Les cheveux peuvent également être mis en forme sans l'aide de moyens extérieurs, simplement avec les doigts.

Avant de procéder à l'étape suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape, facultative, du procédé (étape (ii)), les cheveux imprégnés de la composition réductrice sont donc ensuite rincés soigneusement par une composition aqueuse.

Puis, dans une troisième étape (étape (iii)), on applique sur les cheveux ainsi rincés la composition oxydante de l'invention, dans le but de fixer la nouvelle forme imposée aux cheveux.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions réductrice et oxydante utilisées selon l'invention est de préférence l'eau ou une solution hydroalcoolique d'un alcool inférieur tel que l'éthanol, l'isopropanol ou le butanol.

L'eau oxygénée peut être stabilisée par exemple par la phénacétine, l'acétanilide, les phosphates mono et trisodiques ou par le sulfate d'hydroxy-8 quinoléine, les stannates dont le stannate de sodium.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention (étape (iv)), étape facultative également, les cheveux imprégnés de la composition oxydante sont rincés soigneusement, généralement à l'eau.

On obtient finalement une chevelure facile à démêler, douce. Les cheveux sont ondulés.

La composition oxydante selon l'invention peut également être utilisée dans un procédé de décoloration des fibres kératiniques, et en particulier des cheveux.

Le procédé de décoloration selon l'invention comprend une étape d'application sur les fibres kératiniques d'une composition oxydante selon l'invention, cette composition comprenant de préférence de l'eau oxygénée en milieu alcalin. Classiquement, une deuxième étape du procédé de décoloration selon l'invention est une étape de rinçage des fibres kératiniques.

Des exemples concrets illustrant l'invention vont maintenant être donnés.

Dans ce qui suit ou ce qui précède, sauf mention contraire, les pourcentages sont exprimés en poids.

### EXEMPLE 1 :

La demanderesse a réalisé un test comparatif afin de mettre en évidence l'amélioration apportée au niveau de la résistance mécanique des fibres kératiniques par l'adjonction de céramides dans une composition oxydante de procédé de traitement pour la déformation permanente des fibres kératiniques.

On a réalisé la composition oxydante A, encore appelé fixateur, conforme à l'invention, suivante :

### Fixateur A :

- N-oléyldihydrosphingosine (céramide) 1 %
- eau oxygénée à 200 volumes 4,8 %
- oxyde de lauryl diméthyl amine en solution aqueuse à 30 % MA 1 %
- acide citrique qsp pH=3
- eau déminéralisée qsp 100 %

On a également réalisé une composition oxydante B, comparative, de même composition que A mais ne contenant pas de N-oléyldihydrosphingosine.

Les compositions oxydantes ci-dessus ont été réalisées par simple mélange, après dissolution ou dispersion et chauffage du céramide.

Afin de comparer les deux compositions oxydantes lors d'un traitement de déformation permanente des cheveux, on a réalisé une composition réductrice, de composition suivante :

### Réducteur :

- mélange cocoylamidopropyl bétaïne/monolaurate de glycérol à 30 % MA 1,4 % en l'état
- acide thioglycolique 6,7 %
- bicarbonate d'ammonium 5,1 %
- séquestrant 0,2 %
- ammoniaque à 20 % NH₃ 6,2 %
- eau déminéralisée qsp 100 %

La composition réductrice a été réalisée par simple mélange.

On a ensuite appliqué le réducteur sur des mèches de cheveux sensibilisés, avec un rapport de bain de 2 g / g de cheveux. Par cheveux sensibilisés, on entend des cheveux abîmés à des degrés divers par l'action des agents atmosphériques et/ou de traitements capillaires, mécaniques ou chimiques, tels que des colorations, des décolorations et/ou des permanentes. Après 10 minutes de pose, un rinçage à l'eau a été effectué.

On a ensuite appliqué chacune des compositions A et B sur les cheveux rincés, avec un rapport de bain de 2 g / g de cheveux. Après 5 minutes de pose, les cheveux ont été rincés puis séchés.

L'aptitude de chaque composition à limiter la dégradation de la fibre kératinique a été évaluée selon le protocole suivant : pour chaque chevelure préalablement traitée de la façon indiquée ci-dessus avec la composition A ou B, on a mouillé trois mèches de cheveux qu'on a ensuite disposées sur un support métallique, afin de maintenir les cheveux à la racine. On a ensuite réalisé un brushing de la manière la plus régulière possible.

Les cheveux cassés lors du brushing ont été minutieusement récupérés sur la brosse, introduits dans une boîte de Pétri puis pesés après un conditionnement de 12 heures à une humidité relative de 50% ± 2% et à une température de 20 °C ± 2°C.

Les résultats obtenus sont consignés dans le tableau (I) ci-dessous :

**Tableau (I) :**

| Formule | Quantité de cheveux cassés mg/g |
|---|---|
| Composition A (invention) | 17,8 ± 3,0 |
| Composition B (comparatif) | 28,1 ± 3,2 |

Ces résultats montrent clairement que l'introduction d'un céramide dans une composition oxydante d'un procédé de déformation permanente limite fortement la dégradation de la fibre kératinique.

### EXEMPLE 2 :

Un exemple concret de composition oxydante pour la décoloration des cheveux est donnée ci-dessous :
- persulfate de potassium 27 %
- persulfate de sodium 23 %
- phosphate diammonique 9 %
- silice 15 %
- métasilicate de sodium 15 %
- carbonate de magnésium 6 %
- hydroxyéthlcellulose 2,5 %
- N-oléyldihydrosphongosine 0,5 %
- séquestrant 2 %

## Revendications

1. Composition oxydante, caractérisée par le fait qu'elle comprend, dans un support cosmétique approprié, i) au moins un composé de type céramide et ii) au moins un agent oxydant choisi dans le groupe formé par l'eau oxygénée, les bromates alcalins, les persels ou les polythionates ou leurs mélanges, ledit composé de type céramide étant présent dans la composition à une teneur allant de 0,005 % à 10 % en poids par rapport au poids total de la composition, ladite composition étant exempte de vésicules contenant un agent oxydant.

2. Composition selon la revendication 1, caractérisée par le fait que ladite teneur va de 0,01 % à 5 %.

3. Composition selon la revendication 1 ou 2, caractérisée par le fait que le composé de type céramide est un composé de formule (I) suivante : 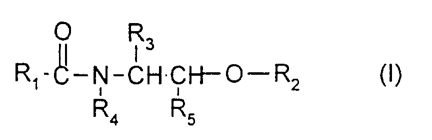 dans laquelle :
- R₁ désigne :
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifiés par un acide gras saturé ou insaturé, linéaire ou ramifié, éventuellement mono ou polyhydroxylé, en C₁-C₃₅,
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent,
- soit un radical R₈-O-CO-(CH2)ₚ, R₈ désignant un radical hydrocarboné en C₁-C₂₀, p étant un entier variant de 1 à 12 ;
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p étant un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

4. Composition selon la revendication 3, caractérisée par le fait que le céramide est un composé de formule (I) pour lesquels R₁ désigne un radical alkyle saturé ou insaturé dérivé d'acides gras en C₁₄-C₂₂ éventuellement hydroxylé; R₂ désigne un atome d'hydrogène ; et R₃ désigne un radical linéaire saturé en C₁₁-C₁₇ éventuellement hydroxylé et de préférence en C₁₃-C₁₅.

5. Composition selon la revendication 4, caractérisée par le fait que le céramide est choisi parmi :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

6. Composition selon la revendication 5, caractérisée par le fait que le céramide est choisi parmi le 2-N-oléoylamino-octadécane-1,3-diol, le 2-N-[2-hydroxypalmitoyl]-amino-octadécane-1,3-diol et la N-stéaroylphytosphingosine.

7. Composition selon l'une quelconque des revendications précédentes, caractérisée par le fait que l'agent oxydant est l'eau oxygénée.

8. Composition selon la revendication 7, caractérisée par le fait que la concentration en eau oxygénée va de 0,5 à 40 volumes.

9. Composition selon la revendication 8, caractérisée par le fait que ladite concentration va de 2 à 30 volumes.

10. Procédé de traitement des matières kératiniques, en particulier des cheveux, en vue d'obtenir une déformation permanente de ces dernières, en particulier sous la forme de cheveux permanentés, ce procédé comprenant les étapes suivantes : (i) on applique sur la matière kératinique à traiter une composition réductrice, la matière kératinique étant mise sous tension mécanique avant, pendant, ou après ladite application, (ii) on rince éventuellement la matière kératinique, (iii) on applique sur la matière kératinique éventuellement rincée une composition oxydante telle que définie à l'une des revendications 1 à 9, (iv) on rince éventuellement à nouveau la matière kératinique.

11. Procédé de décoloration des matières kératiniques, en particulier des cheveux, comprenant les étapes suivantes : i) on applique sur la matière kératinique une composition oxydante selon l'une quelconque des revendications 1 à 9, ii) on rince la matière kératinique ainsi traitée.

## Patentansprüche

1. Oxidierende Zusammensetzung, dadurch gekennzeichnet, dass sie in einem geeigneten kosmetischen Träger (i) mindestens eine Verbindung vom Ceramidtyp und (ii) mindestens ein Oxidationsmittel enthält, das unter Wasserstoffperoxid, Alkalibromaten, Salzen von Persäuren oder Polythionaten oder den Gemischen dieser Verbindungen ausgewählt ist, wobei die Verbindung vom Ceramidtyp in der Zusammensetzung in einem Mengenanteil von 0,005 bis 10 Gew.-%, bezogen auf das Gesamtgewicht der Zusammensetzung, vorliegt und wobei die Zusammensetzung frei von Vesikeln ist, die ein Oxidationsmittel enthalten.

2. Zusammensetzung nach Anspruch 1, dadurch gekennzeichnet, dass der Mengenanteil im Bereich von 0,01 bis 5 % liegt.

3. Zusammensetzung nach Anspruch 1 oder 2, dadurch gekennzeichnet, dass die Verbindung vom Ceramidtyp eine Verbindung der folgenden Formel (I) ist: 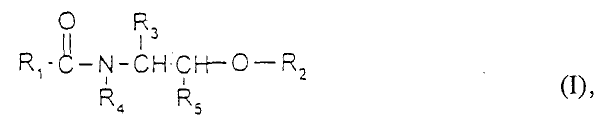 worin:
- R₁
- entweder eine gesättigte oder ungesättigte, geradkettige oder verzweigte Kohlenwasserstoffgruppe mit 1 bis 50 Kohlenstoffatomen, vorzugsweise mit 5 bis 50 Kohlenstoffatomen, wobei die Gruppe mit einer oder mehreren Hydroxygruppen substituiert sein kann, die gegebenenfalls mit einer Säure R₇COOH verestert sind, worin R₇ eine gegebenenfalls mono- oder polyhydroxylierte, gesättigte oder ungesättigte, geradkettige oder verzweigte Kohlenwasserstoffgruppe mit 1 bis 35 Kohlenstoffatomen bedeutet, wobei die Hydroxygruppe oder die Hydroxygruppen der Gruppe R₇ mit einer gegebenenfalls mono- oder polyhydroxylierten, geradkettigen oder verzweigten, gesättigten oder ungesättigten Fettsäure mit 1 bis 35 Kohlenstoffatomen verestert sein können,
- oder eine Gruppe R"-(NR-CO)-R', worin R ein Wasserstoffatom oder eine mono- oder polyhydroxylierte C₁₋₂₀-Kohlenwasserstoffgruppe, vorzugsweise eine monohydroxylierte C₁₋₂₀-Kohlenwasserstoffgruppe, bedeutet und R' und R" Kohlenwasserstoffgruppen sind, deren Summe der Kohlenstoffatome im Bereich von 9 bis 30 liegt, wobei R' eine zweiwertige Gruppe ist,
- oder eine Gruppe R₈-O-CO-(CH₂)ₚ bedeutet, worin R₈ eine Kohlenwasserstoffgruppe mit 1 bis 20 Kohlenstoffatomen und p eine ganze Zahl im Bereich von 1 bis 12 bedeuten;
- R₂ unter einem Wasserstoffatom, einer Gruppe vom Saccharidtyp, insbesondere einer Gruppe (Glykosyl)ₙ, (Galactosyl)ₘ oder Sulfogalactosyl, einem Sulfatrest oder einem Phosphatrest, einer Phosphorylethylamin-Gruppe und einer Phosphorylethylammonium-Gruppe ausgewählt ist, worin n eine ganze Zahl im Bereich von 1 bis 4 und m einen ganze Zahl im Bereich von 1 bis 8 bedeuten;
- R₃ ein Wasserstoffatom oder eine gegebenenfalls hydroxylierte, gesättigte oder ungesättigte Kohlenwasserstoffgruppe mit 1 bis 33 Kohlenstoffatomen bedeutet, wobei die Hydroxygruppe oder die Hydroxygruppen mit einer anorganischen Säure oder einer Säure R₇COOH verestert sein können, worin R₇ die gleichen Bedeutungen wie oben aufweist, wobei die Hydroxygruppe oder die Hydroxygruppen mit einer Gruppe (Glykosyl)ₙ, (Galactosyl)ₘ, Sulfogalactosyl, Phosphorylethylamin oder Phosphorylethylammonium verethert sein können, wobei R₃ ferner mit einer oder mehreren C₁₋₁₄-Alkylgruppen substituiert sein kann;
- R₄ ein Wasserstoffatom, eine Methylgruppe, eine Ethylgruppe, eine gegebenenfalls hydroxylierte, geradkettige oder verzweigte, gesättigte oder ungesättigte Kohlenwasserstoffgruppe mit 3 bis 50 Kohlenstoffatomen oder eine Gruppe -CH₂-CHOH-CH₂-O-R₆ bedeutet, worin R₆ eine Kohlenwasserstoffgruppe mit 10 bis 26 Kohlenstoffatomen oder eine Gruppe R₈-O-CO-(CH₂)ₚ bedeutet, worin R₈ eine Kohlenwasserstoffgruppe mit 1 bis 20 Kohlenstoffatomen bedeutet und p eine ganze Zahl im Bereich von 1 bis 12 ist; und
- R₅ ein Wasserstoffatom oder eine gegebenenfalls mono- oder polyhydroxylierte, geradkettige oder verzweigte, gesättigte oder ungesättigte C₁₋₃₀-Kohlenwasserstoffgruppe bedeutet, wobei die Hydroxygruppe oder die Hydroxygruppen mit einer Gruppe (Glykosyl)ₙ, (Galactosyl)ₘ, Sulfogalactosyl, Phosphorylethylamin oder Phosphorylethylammonium verethert sein können,
mit der Maßgabe, dass R₃ und R₅ Wasserstoff bedeuten oder dass R₄ nicht Wasserstoff, Methyl oder Ethyl bedeutet, wenn R₃ Wasserstoff und R₅ Methyl bedeuten.

4. Zusammensetzung nach Anspruch 3, dadurch gekennzeichnet, dass das Ceramid eine Verbindung der Formel (I) ist, worin bedeuten:
- R₁ eine gesättigte oder ungesättigte Gruppe, die von einer C₁₄₋₂₂-Fettsäure abgeleitet ist, die gegebenenfalls hydroxyliert ist,
- R₂ ein Wasserstoffatom und
- R₃ eine gesättigte, geradkettige Gruppe mit 11 bis 17 Kohlenstoffatomen und vorzugsweise mit 13 bis 15 Kohlenstoffatomen, die gegebenenfalls hydroxyliert ist.

5. Zusammensetzung nach Anspruch 4, dadurch gekennzeichnet, dass das Ceramid ausgewählt ist unter:
- 2-N-Linoleoylamino-octadecan-1,3-diol,
- 2-N-Oleoylamino-octadecan-1,3-diol,
- 2-N-Palmitoylamino-octadecan-1,3-diol,
- 2-N-Stearoylamino-octadecan-1,3-diol,
- 2-N-Behenoylamino-octadecan-1,3-diol,
- 2-N-[2-Hydroxy-palmitoyl]-amino-octadecan-1,3-diol,
- 2-N-Stearoylamino-octadecan-1,3,4-triol und insbesondere N-Stearoylphytosphingosin,
- 2-N-Palmitoylamino-hexadecan-1,3-diol
oder den Gemischen dieser Verbindungen.

6. Zusammensetzung nach Anspruch 5, dadurch gekennzeichnet, dass das Ceramid unter 2-N-Oleoylamino-octadecan-1,3-diol, 2-N-[2-Hydroxy-palmitoyl]-amino-octadecan-1,3-diol und N-Stearoylphytosphingosin ausgewählt ist.

7. Zusammensetzung nach einem der vorhergehenden Ansprüche, dadurch gekennzeichnet, dass das Oxidationsmittel Wasserstoffperoxid ist.

8. Zusammensetzung nach Anspruch 7, dadurch gekennzeichnet, dass die Wasserstoffperoxid-Konzentration im Bereich von 0,5 bis 40 Volumina liegt.

9. Zusammensetzung nach Anspruch 8, dadurch gekennzeichnet, dass die Konzentration im Bereich von 2 bis 30 Volumina liegt.

10. Verfahren zur Behandlung keratinischer Materialien und insbesondere des Haars, um eine dauerhafte Verformung des Haars, insbesondere in Form von dauergewelltem Haar, zu erzielen, wobei das Verfahren die folgenden Schritte umfasst:
(i) Auf das zu behandelnde keratinische Material wird eine reduzierende Zusammensetzung aufgebracht, wobei das keratinische Material vor, während oder nach dem Aufbringen unter mechanische Spannung gesetzt wird,
(ii) das keratinische Material wird gegebenenfalls gespült,
(iii) auf das gegebenenfalls gespülte keratinische Material wird eine in einem der Ansprüche 1 bis 9 definierte oxidierende Zusammensetzung aufgebracht und
(iv) das keratinische Material wird gegebenenfalls nochmals gespült.

11. Verfahren zum Entfärben keratinischer Materialien und insbesondere des Haars, das die folgenden Schritte umfasst:
(i) Auf das zu behandelnde keratinische Material wird eine in einem der Ansprüche 1 bis 9 definierte oxidierende Zusammensetzung aufgebracht und
(ii) das so behandelte keratinische Material wird gespült.

## Claims

1. Oxidizing composition, characterized in that it comprises, in a suitable cosmetic vehicle, i) at least one ceramide-type compound and ii) at least one oxidizing agent chosen from the group formed by aqueous hydrogen peroxide solution, alkaline bromates, persalts and polythionates or mixtures thereof, the said ceramide-type compound being present in the composition at a content ranging from 0.005% to 10% by weight relative to the total weight of the composition, the said composition being free of vesicles containing an oxidizing agent.

2. Composition according to Claim 1,
characterized in that the said content ranges from 0.01% to 5%.

3. Composition according to Claim 1 or 2,
characterized in that the ceramide-type compound is a compound of formula (I) below: 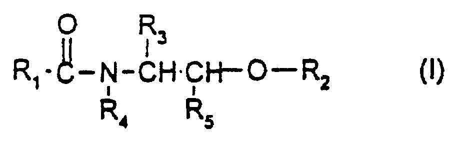 in which:
- R₁ denotes:
- either a linear or branched, saturated or unsaturated C₁-C₅₀, preferably C₅-C₅₀, hydrocarbon radical, it being possible for this radical to be substituted with one or more hydroxyl groups that are optionally esterified with an acid R₇COOH, R₇ being a saturated or unsaturated, linear or branched, optionally mono- or polyhydroxylated, C₁-C₃₅ hydrocarbon radical, it being possible for the hydroxyl(s) of the radical R₇ to be esterified with a saturated or unsaturated, linear or branched, optionally mono- or polyhydroxylated, C₁-C₃₅ fatty acid,
- or a radical R"-(NR-CO)-R', R denotes a hydrogen atom or a mono- or polyhydroxylated, preferably monohydroxylated, C₁-C₂₀ hydrocarbon radical, R' and R" are hydrocarbon radicals the sum of whose carbon atoms is between 9 and 30, R' being a divalent radical,
- or a radical R₈-O-CO-(CH₂)ₚ, R₈ denoting a C₁-C₂₀ hydrocarbon radical, p being an integer ranging from 1 to 12;
- R₂ is chosen from a hydrogen atom, a radical of saccharide type, in particular a (glycosyl)ₙ, (galactosyl)ₘ or sulphogalactosyl radical, a sulphate or phosphate residue, a phosphorylethylamine radical and a phosphorylethylammonium radical, in which n is an integer ranging from 1 to 4 and m is an integer ranging from 1 to 8;
- R₃ denotes a hydrogen atom or a saturated or unsaturated, hydroxylated or non-hydroxylated C₁-C₃₃ hydrocarbon radical, it being possible for the hydroxyl(s) to be esterified with an inorganic acid or an acid R₇COOH, R₇ having the same meanings as above, it being possible for the hydroxyl(s) to be etherified with a (glycosyl)ₙ, (galactosyl)ₘ, sulphogalactosyl, phosphorylethylamine or phosphorylethylammonium radical, it also being possible for R₃ to be substituted with one or more C₁-C₁₄ alkyl radicals;
- R₄ denotes a hydrogen atom, a methyl or ethyl radical, a saturated or unsaturated, linear or branched, optionally hydroxylated C₃-C₅₀ hydrocarbon radical or a radical -CH₂-CHOH-CH₂-O-R₆ in which R₆ denotes a C₁₀-C₂₆ hydrocarbon radical or a radical R₈-O-CO-(CH₂)ₚ, R₈ denotes a C₁-C₂₀ hydrocarbon radical, p being an integer ranging from 1 to 12,
- R₅ denotes a hydrogen atom or a saturated or unsaturated, linear or branched, optionally mono- or polyhydroxylated C₁-C₃₀ hydrocarbon radical, it being possible for the hydroxyl(s) to be etherified with a (glycosyl)ₙ, (galactosyl)ₘ, sulphogalactosyl, phosphorylethylamine or phosphorylethylammonium radical,
with the proviso that when R₃ and R₅ denote hydrogen or when R₃ denotes hydrogen and R₅ denotes methyl, then R₄ does not denote a hydrogen atom or a methyl or ethyl radical.

4. Composition according to Claim 3,
characterized in that the ceramide is a compound of formula (I) for which R₁ denotes a saturated or unsaturated, optionally hydroxylated alkyl radical derived from C₁₄-C₂₂ fatty acids; R₂ denotes a hydrogen atom; and R₃ denotes a saturated, linear, optionally hydroxylated C₁₁-C₁₇ and preferably C₁₃-C₁₅ radical.

5. Composition according to Claim 4,
characterized in that the ceramide is chosen from:
- 2-N-linoleoylaminooctadecane-1,3-diol,
- 2-N-oleoylaminooctadecane-1,3-diol,
- 2-N-palmitoylaminooctadecane-1,3-diol,
- 2-N-stearoylaminooctadecane-1,3-diol,
- 2-N-behenoylaminooctadecane-1,3-diol,
- 2-N-[2-hydroxypalmitoyl]aminooctadecane-1,3-diol,
- 2-N-stearoylaminooctadecane-1,3,4-triol and in particular N-stearoylphytosphingosine,
- 2-N-palmitoylaminohexadecane-1,3-diol
or mixtures of these compounds.

6. Composition according to Claim 5,
characterized in that the ceramide is chosen from 2-N-oleoylaminooctadecane-1,3-diol, 2-N-[2-hydroxypalmitoyl]aminooctadecane-1,3-diol and N-stearoylphytosphingosine.

7. Composition according to any one of the preceding claims, characterized in that the oxidizing agent is aqueous hydrogen peroxide solution.

8. Composition according to Claim 7,
characterized in that the aqueous hydrogen peroxide solution concentration ranges from 0.5 to 40 volumes.

9. Composition according to Claim 8,
characterized in that the said concentration ranges from 2 to 30 volumes.

10. Process for treating keratin substances, in particular the hair, in order to obtain permanent reshaping of this hair, in particular in the form of permanent-waved hair, this process comprising the following steps: (i) a reducing composition is applied to the keratin substance to be treated, the keratin substance being placed under mechanical tension before, during or after the said application, (ii) the keratin substance is optionally rinsed, (iii) an oxidizing composition as defined in one of Claims 1 to 9 is applied to the optionally rinsed keratin substance, (iv) the keratin substance is optionally rinsed again.

11. Process for bleaching keratin substances, in particular the hair, comprising the following steps: i) an oxidizing composition according to any one of Claims 1 to 9 is applied to the keratin substance, ii) the keratin substance thus treated is rinsed.
